# EUROPEAN PATENT APPLICATION

(11) **EP 2 810 623 A2**
(43) Date of publication of application: **10.12.2014**
(21) Application number: 14153162.4
(22) Date of filing: 30.01.2014
(51) Int. Cl.: A61F 2/966

(54) **Pusher guidewire**

(30) Priority: 05.06.2013 JP 2013118988
(71) Applicant: ASAHI INTECC CO., LTD., Nagoya-shi, Aichi 463-0024 (JP)
(72) Inventor: Nishigishi, Makoto, Nagoya-shi, Aichi 463-0024 (JP)
(74) Representative: Winter, Brandl, Fürniss, Hübner, Röss, Kaiser, Polte - Partnerschaft mbB

(57) **Abstract**

[Object]

It is an object of the present invention to provide a pusher guidewire in which a coil has a free rear end that is not fixed to a core shaft, thereby allowing a stent that has been partially deployed from the front end of a catheter to be retrieved into the catheter.

[Solution]

In a pusher guidewire (1, 1a, 1b, 1c, 1d), a front end coil (14, 64, 74, 84, 94) has a free rear end (14a, 64a, 74a, 84a, 94a) that is not fixed to a first front end portion (12a) of a core shaft (12). By pulling the core shaft (12) toward the rear end, the free rear end (14a, 64a, 74a, 84a, 94a) catches a gap (10a) of a partially-deployed stent (10). By rotating the core shaft (12) in this state, a front end portion (11) of the stent (10) deployed from a front end opening (24) of a catheter (2) is wrapped around the outer circumference of the front end coil (14, 64, 74, 84, 94). Further pulling the core shaft (12) toward the rear end with the wrapped stent (10) retrieves the partially-deployed stent (10) into the catheter (2), thereby preventing the stent (10) from damaging the inner wall of a normal blood vessel.

## Description

### [Technical Field]

The present invention relates to a pusher guidewire that delivers a stent housed in a catheter to a target site.

### [Background Art]

A stent is a medical device that supports the lumens of blood vessels and digestive organs so as to prevent re-constriction of blood vessels and digestive organs expanded by a balloon catheter and the like. Stents are broadly classified into two types: balloon expandable stents that are expanded by a balloon catheter and the like; and self-expandable stents that naturally expand on their own. In recent years, self-expandable stents that do not easily deform even under an external force are often used.

One known method for delivering a self-expandable stent to a target site is to house the stent in a front end portion of a catheter with the stent arranged around the outer circumference of a pusher guidewire, advance the catheter to the target site, and then push the pusher guidewire toward the front end so as to deploy the stent from the front end of the catheter at the target site (see, for example, Patent Literature 1 mentioned below).

However, with the aforementioned pusher guidewire, if a doctor notices that the deployment position of the stent has shifted away from the target site in the midst of deployment of the stent from the front end of the catheter, the doctor can do nothing but pull the pusher guidewire toward the rear end so as to retrieve the stent in the state where the stent is pinched between the pusher guidewire and the catheter. Once a self-expandable stent has been deployed from the front end of a catheter, it cannot be retrieved into the catheter. For this reason, when a doctor attempts to retrieve the deployed stent, there is a risk of damaging the inner wall of a normal blood vessel by the deployed stent.

### [Citation List]

### [Patent Literature]

[Patent Literature 1] Japanese Patent No. 4498709

### [Summary of Invention]

### [Technical Problem]

The present invention has been made in view of the above issue. An object of the present invention is to provide a pusher guidewire in which a coil has a free rear end that is not fixed to a core shaft, thereby allowing a stent that has been partially deployed from the front end of a catheter to be retrieved into the catheter.

### [Solution to Problem]

The above object is achieved by the means listed below.

A first aspect of the present invention is a pusher guidewire for delivering a stent to a target site, including: a core shaft; a coil covering a front end portion of the core shaft; and a joining member joining the front end portion of the core shaft and the coil, characterized in that a rear end of the coil is a free end that is not fixed to the core shaft.

A second aspect of the present invention is the pusher guidewire according to the first aspect, characterized in that an outer diameter of the coil is larger from the joining member to the rear end of the coil than from a front end of the coil to the joining member.

A third aspect of the present invention is the pusher guidewire according to the first or second aspect, characterized in that a wire diameter of a wire constituting the coil is larger from the joining member to the rear end of the coil than from a front end of the coil to the joining member.

A fourth aspect of the present invention is the pusher guidewire according to any of the first to third aspects, characterized in that a winding pitch of the coil is larger from the joining member to the rear end of the coil than from a front end of the coil to the joining member.

A fifth aspect of the present invention is the pusher guidewire according to any of the first to fourth aspects, characterized in that the rear end of the coil is provided with a latching member.

### [Advantageous Effects of Invention]

In the pusher guidewire according to the first aspect of the present invention, the rear end of the coil is a free end that is not fixed to the core shaft. Therefore, by pulling the core shaft toward the rear end, the free rear end of the coil catches a gap of the partially-deployed stent. By rotating the core shaft in this state, the stent deployed from the front end of the catheter can be wrapped around the outer circumference of the coil. By further pulling the core shaft toward the rear end with the stent thus wrapped, the partially-deployed stent can be retrieved into the catheter. As a result, the inner wall of a normal blood vessel can be prevented from being damaged by the stent.

In the pusher guidewire according to the second aspect of the present invention, the outer diameter of the coil is larger from the joining member to the rear end of the coil than from the front end of the coil to the joining member. Reducing the outer diameter of the coil on the front end side allows the pusher guidewire to reach blood vessels at more extreme ends. Furthermore, the gaps of the stent are not expandable and hence narrow when the stent is housed in the catheter, but are widened once the stent has been deployed from the front end of the catheter. Therefore, increasing the outer diameter of the coil on the rear end side makes it easy for the rear end of the coil to catch a gap of the stent that is starting to expand. In this way, the partially-deployed stent can be retrieved into the catheter more reliably.

In the pusher guidewire according to the third aspect of the present invention, the wire diameter of the wire constituting the coil is larger from the joining member to the rear end of the coil than from the front end of the coil to the joining member. Reducing the wire diameter of the wire constituting the coil on the front end side reduces the outer diameter of the coil on the front end side, thereby allowing the pusher guidewire to reach blood vessels at more extreme ends. Furthermore, increasing the wire diameter of the wire constituting the coil on the rear end side increases the force by which the coil on the rear end side restrains the stent, thereby making it easy to wrap the stent deployed from the front end of the catheter around the outer circumference of the coil. In this way, the partially-deployed stent can be retrieved into the catheter more reliably.

In the pusher guidewire according to the fourth aspect of the present invention, the winding pitch of the coil is larger from the joining member to the rear end of the coil than from the front end of the coil to the joining member. By narrowing the winding pitch of the coil on the front end side, rotation of the core shaft can be transmitted to the front end of the coil via the joining member. On the other hand, by widening the winding pitch of the coil on the rear end side, the flexibility of the free rear end of the coil can be increased, and a clearance can be provided between portions of the wire constituting the coil. As a result, the flexibility is improved, and the rear end of the coil, in which the clearance is provided between portions of the wire, easily catches a gap of the stent In this way, the partially-deployed stent can be retrieved into the catheter more reliably.

In the pusher guidewire according to the fifth aspect of the present invention, the rear end of the coil is provided with a latching member. The latching member has a hook-like shape or a bulged shape. This can reduce the risk that the rear end of the coil that caught a gap of the stent slips off the gap of the stent when the core shaft is rotated.

### [Brief Description of Drawings]

Fig. 1A is an overall view showing a pusher guidewire according to an embodiment in the state where a stent is housed in a catheter. Fig. 1B is a cross-sectional view of Fig. 1A.
Fig. 2A is an overall view showing the pusher guidewire according to the embodiment in the state where the stent is in the midst of deployment from the front end of the catheter. Fig. 2B is a cross-sectional view of Fig. 2A.
Fig. 3A is an overall view showing the pusher guidewire according to the embodiment in the state where the rear end of a coil has caught a gap of the stent Fig. 3B is a cross-sectional view of Fig. 3A.
Fig. 4A is an overall view of the pusher guidewire according to the embodiment in the state where the stent deployed from the front end of the catheter has been wrapped around the outer circumference of the coil as a result of rotating a core shaft. Fig. 4B is a cross-sectional view of Fig. 4A.
Fig. 5A is an overall view showing the pusher guidewire according to the embodiment in the state where the stent has been retrieved into the catheter while being wrapped around the outer circumference of the coil. Fig. 5B is a cross-sectional view of Fig. 5A.
Fig. 6 is a cross-sectional view showing a pusher guidewire according to a second embodiment, which is a modification example of Fig. 2B.
Fig. 7 is a cross-sectional view showing a pusher guidewire according to a third embodiment, which is a modification example of Fig. 2B.
Fig. 8 is a cross-sectional view showing a pusher guidewire according to a fourth embodiment, which is a modification example of Fig. 2B.
Fig. 9 is an overall view showing a pusher guidewire according to a fifth embodiment, which is a modification example of Fig. 2A.

### [Description of Embodiments]

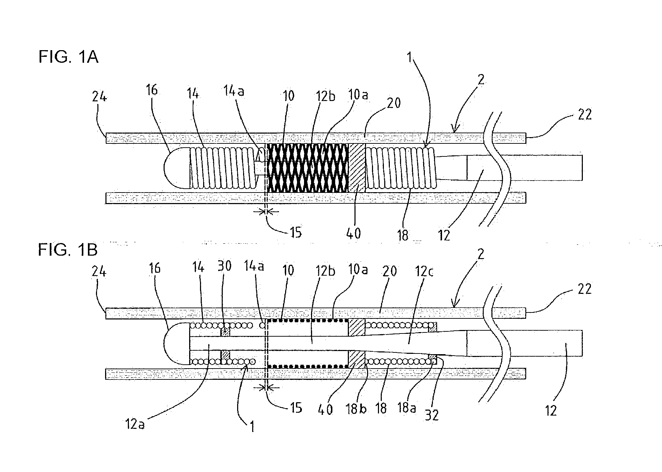

A pusher guidewire 1 according to the present embodiment will now be described with reference to Figs. 1A to 5B. It should be noted that, in Figs. 1A to 5B, the left side corresponds to the front end side (distal side) inserted into a body, and the right side corresponds to the rear end side (proximal side, base end side) operated by an operator such as a doctor. Figs. 1B, 2B, 3B, 4B and 5B are cross-sectional views of Figs. 1A, 2A, 3A, 4A and 5A, respectively.

First, as shown in Figs. 1A and 1B, the pusher guidewire 1 for delivering a stent 10 to a target site includes a core shaft 12, a front end coil 14, a front end tip 16, and a rear end coil 18. The front end coil 14 is wound around the outer circumference of a first front end portion 12a of the core shaft 12. The front end tip 16 joins the front end of the core shaft 12 and the front end of the front end coil 14. The rear end coil 18 is arranged on the rear end side (the light side of Figs. 1A and 1B) of the front end coil 14 and wound around the outer circumference of a third front end portion 12c of the core shaft 12. For the sake of convenience, the core shaft 12 will be described as including the following three portions: the first front end portion 12a covered by the front end coil 14; the third front end portion 12c covered by the rear end coil 18; and a second front end portion 12b, which is positioned between the first front end portion 12a and the third front end portion 12c and around which the stent 10 is arranged.

A catheter 2 has a tubular body 20 into which the pusher guidewire 1 can be inserted. The pusher guidewire 1, around which the stent 10 is arranged between the front end coil 14 and the rear end coil 18, can be inserted from a rear end opening 22 of the catheter 2. A description of the catheter 2 is omitted as a conventional catheter can be used as the catheter 2.

As shown in Fig. 1B, the front end coil 14 is joined to the first front end portion 12a of the core shaft 12 by a joining member 30 (for example, brazing filler material). Similarly, a rear end 18a of the rear end coil 18 is joined to the third front end portion 12c of the core shaft 12 by a joining member 32 (for example, brazing filler material).

A pusher 40, which is joined to the second front end portion 12b of the core shaft 12, is provided between the stent 10 and the rear end coil 18. The pusher 40 is constituted by a tubular body made of metal such as stainless steel, and is used to deploy the stent 10 from a front end opening 24 of the catheter 2 when the doctor pushes the core shaft 12 toward the front end

(the left side of Figs. 1A and 1B). A front end 18b of the rear end coil 18 is joined to the pusher 40.

As shown in Figs. 1A and 1B, a rear end 14a of the front end coil 14 is a free end that is not fixed to the first front end portion 12a of the core shaft 12. Furthermore, there is a clearance 15 between the rear end 14a of the front end coil 14 and the front end of the stent 10. Therefore, as will be described later, the presence of the clearance 15 prevents the rear end 14a of the front end coil 14 from catching a gap 10a of the stent 10 when the pusher guidewire 1 is pushed toward the front end (the left side of Figs. 1A and 1B). On the other hand, when the pusher guidewire 1 is pulled toward the rear end (the right side of Figs. 1A and 1B), the rear end 14a of the front end coil 14 can catch the gap 10a of the stent 10.

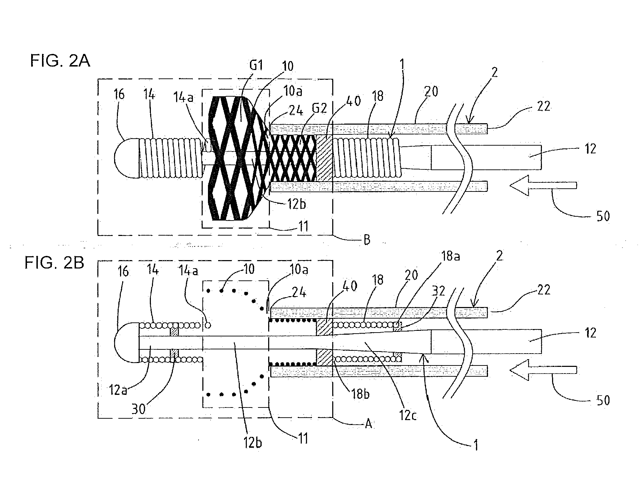

Once the catheter 2 has been advanced to the target site, the doctor pushes the core shaft 12 toward the front end (the direction of arrow 50) so as to deploy the stent 10 housed in the catheter 2 at the target site (see Figs. 2A and 2B). In this way, a front end portion 11 of the stent 10 is deployed from the front end opening 24 of the catheter 2 at the target site. The gaps 10a of the stent 10 are not expandable and hence narrow (G2) when the stent 10 is housed in the catheter 2, but are widened (G1) once the stent 1 has been deployed from the front end opening 24 of the catheter 2. That is to say, the gaps 10a of the stent 10 change from the narrow state G2 to the widened state G1 (see Fig. 2A).

The catheter 2 is fixed such that it does not move when the stent 10 is deployed at the target site. However, in the case where the target site is in a curved peripheral blood vessel and hence a front end portion of the catheter 2 is curved, if the core shaft 12 is forcefully pushed toward the front end (the left side of Figs. 2A and 2B), the front end opening 24 of the catheter 2 may move in the same direction (toward the left side of Figs. 2A and 2B), possibly shifting the deployment position of the stent 10 away from the target site. The shift of the deployment position of the stent 10 away from the target site may be noticed in the midst of deployment of the stent 10 from the front end opening 24 of the catheter 2.

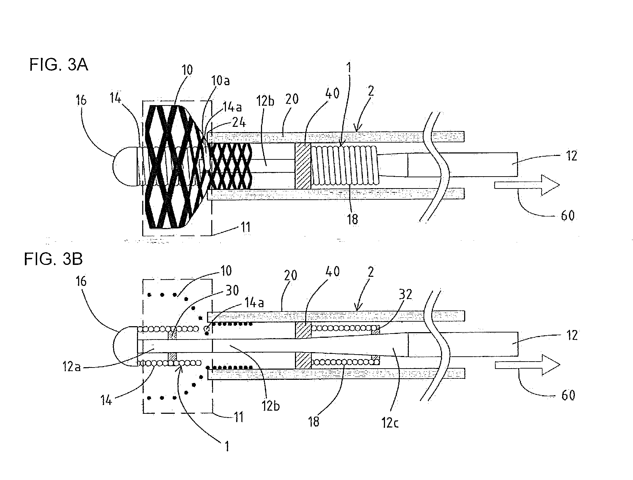

In the pusher guidewire 1 according to the present embodiment, the rear end 14a of the front end coil 14 is a free end that is not fixed to the first front end portion 12a of the core shaft 12, and therefore pulling the core shaft 12 toward the rear end (the direction of arrow 60) allows the rear end 14a of the front end coil 14 to catch a gap 10a of the stent 10 (see Figs. 3A and 3B).

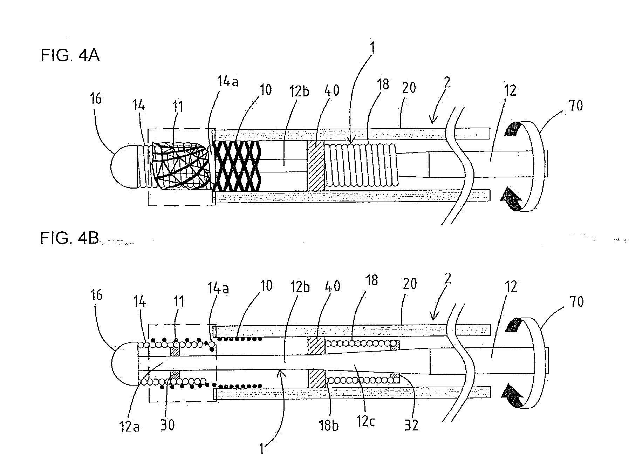

By rotating the core shaft 12 in the circumferential direction (the direction of arrow 70) with the rear end 14a of the front end coil 14 catching the gap 10a of the stent 10, the front end portion 11 of the stent 10 deployed from the front end opening 24 of the catheter 2 can be wrapped around the outer circumference of the front end coil 14 (see Figs. 4A and 4B).

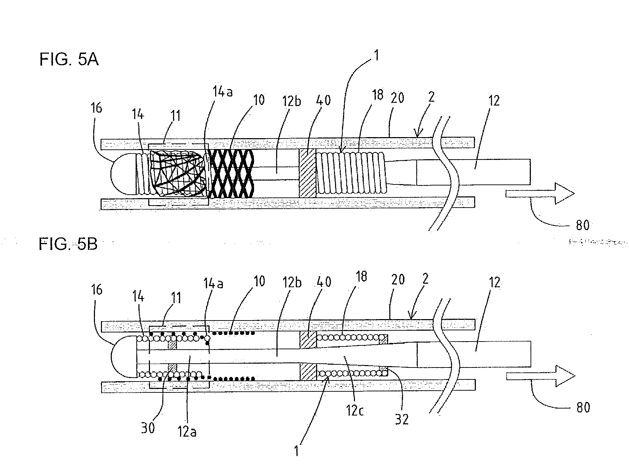

By further pulling the core shaft 12 toward the rear end (the direction of arrow 80) with the front end portion 11 of the stent 10 wrapped around the outer circumference of the front end coil 14, the partially-deployed stent 10 can be retrieved into the catheter 2 (see Figs. 5A and 5B).

By using the free rear end 14a of the front end coil 14 in the above manner, the stent 10 deployed from the front end opening 24 of the catheter 2 can be retrieved into the catheter 2. Therefore, when the doctor attempts to retrieve the stent 10 after noticing the shift of the deployment position of the stent 10 away from the target site, a blood vessel can be prevented from being damaged by the stent 10 deployed from the front end opening 24 of the catheter 2.

The following describes the materials of the constituent elements of the pusher guidewire 1 according to the present embodiment. It should be noted, however, that these constituent elements are not particularly limited to being made of the following materials.

The core shaft 12 may be formed by stainless steel (e.g., SUS 304 and SUS 316) or superelastic alloy such as Ni-Ti alloy.

The front end coil 14 and the rear end coil 18 may be formed by radiopaque wires. Such wires are made of, for example, gold, platinum, tungsten, or alloy containing these chemical elements. With the front end coil 14 and the rear end coil 18 formed by radiopaque wires, the operator can acknowledge the positions of the front end coil 14 and the rear end coil 18 on radiographic images.

The front end coil 14 and the rear end coil 18 may each be formed by a single wire or by a strand made by stranding a plurality of wires. Preferably, the front end coil 14 and the rear end coil 18 are each formed by a strand as it has superior characteristics compared to a single wire in terms of flexibility and resilience.

The front end tip 16 may be formed by a radiopaque material, such as gold, platinum, tungsten, or alloy containing these chemical elements, so that the position of the front end of the pusher guidewire 1 can be acknowledged on radiographic images.

The joining members 30, 32 may be formed by a brazing filler material (e.g., aluminum alloy, silver, and gold), metal solder (e.g., Au-Sn alloy), and the like.

Similarly to the core shaft 12, the pusher 40 may be formed by stainless steel (e.g., SUS 304 and SUS 316) or superelastic alloy such as Ni-Ti alloy.

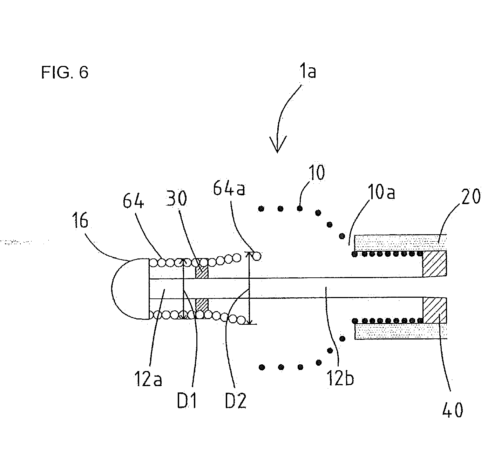

A pusher guidewire 1a according to a second embodiment will now be described with reference to Fig. 6. It should be noted that, in Fig. 6, similarly to Figs. 1A to 5B, the left side corresponds to the front end side (distal side) inserted into a body, and the right side corresponds to the rear end side (proximal side, base end side) operated by an operator such as a doctor. Fig. 6 is an enlarged cross-sectional view of section A shown in Fig. 2B.

The pusher guidewire 1a is different from the pusher guidewire 1 shown in Figs. 1A to 5B in that the outer diameter of a front end coil 64 is larger from the joining member 30 to a rear end 64a of the front end coil 64 (D2) than from the front end of the front end coil 64 to the joining member 30 (D1) (D2 > D1). Reducing the outer diameter D1 of the front end coil 64 on the front end side in this manner allows the pusher guidewire 1a to reach blood vessels at more extreme ends. Furthermore, as described earlier, the gaps 10a of the stent 10 are not expandable and hence narrow (G2) when the stent 10 is housed in the catheter 2, but are widened (G1) once the stent 10 has been deployed from the front end opening 24 of the catheter 2 (see Figs. 2A). Therefore, increasing the outer diameter D2 of the front end coil 64 on the rear end side makes it easy for the rear end 64a of the front end coil 64 to catch a gap 10a of the stent 10 that is starting to expand. Consequently, the partially-deployed stent 10 can be retrieved into the catheter 2 more reliably.

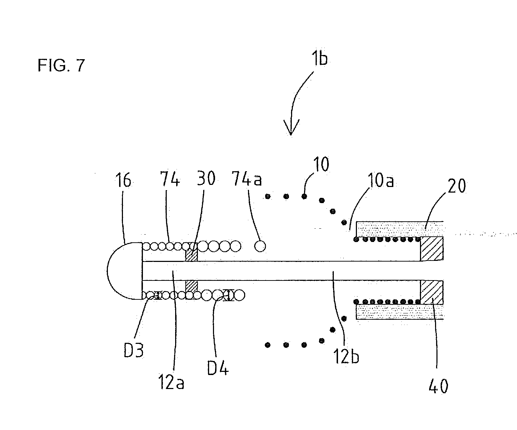

A pusher guidewire 1b according to a third embodiment will now be described with reference to Fig. 7. It should be noted that, in Fig. 7, similarly to Figs. 1A to 5B, the left side corresponds to the front end side (distal side) inserted into a body, and the right side corresponds to the rear end side (proximal side, base end side) operated by an operator such as a doctor. Fig. 7 is an enlarged cross-sectional view of section A shown in Fig. 2B.

The pusher guidewire 16 is different from the pusher guidewire 1 shown in Figs. 1A to 5B in that the wire diameter of a wire constituting a front end coil 74 is larger from the joining member 30 to a rear end 74a of the front end coil 74 (D4) than from the front end of the front end coil 74 to the joining member 30 (D3) (D3 < D4). Reducing the wire diameter D3 of the wire constituting the front end coil 74 on the front end side reduces the outer diameter of the front end coil 74 on the front end side, thereby allowing the pusher guidewire 1b to reach blood vessels at more extreme ends. Furthermore, increasing the wire diameter D4 of the wire constituting the front end coil 74 on the rear end side increases the force by which the front end coil 74 on the rear end side restrains the stent 10, thereby making it easy to wrap the stent 10 deployed from the front end opening 24 of the catheter 2 around the outer circumference of the front end coil 74.

The wire diameter D3 of the front end coil 74 on the front end side may be reduced as follows: after forming the front end coil 74 by winding a wire having the large wire diameter D4 around the outer circumference of the first front end portion 12a of the core shaft 12, electropolishing is applied to the front end coil 74 between the front end thereof and the joining member 30 so as to transform the wire diameter of the front end coil 74 on the front end side from the large wire diameter D4 to the small wire diameter D3. Alternatively, after forming the front end coil 74 on the front end side by winding a wire having the small wire diameter D3 around the outer circumference of the first front end portion 12a of the core shaft 12, a separate wire having the wire diameter D4, which is larger than the wire diameter D3 of the front end coil 74 on the front end side, may be wound so as to form the front end coil 74 on the rear end side.

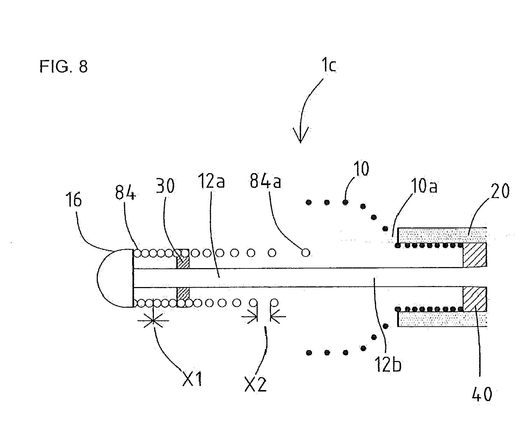

A pusher guidewire 1c according to a fourth embodiment will now be described with reference to Fig. 8. It should be noted that, in Fig. 8, similarly to Figs. 1A to 5B, the left side corresponds to the front end side (distal side) inserted into a body, and the right side corresponds to the rear end side (proximal side, base end side) operated by an operator such as a doctor. Fig. 8 is an enlarged cross-sectional view of section A shown in Fig. 2B.

The pusher guidewire 1c is different from the pusher guidewire 1 shown in Figs. 1A to 5B in that the winding pitch of a front end coil 84 is larger from the joining member 30 to a rear end 84a of the front end coil 84 (X2) than from the front end of the front end coil 84 to the joining member 30 (X1). By narrowing the winding pitch X1 of the front end coil 84 on the front end side, rotation of the core shaft 12 can be transmitted to the front end of the front end coil 84 via the joining member 30. On the other hand, by widening the winding pitch X2 of the front end coil 84 on the rear end side, the flexibility of the free rear end 84a of the front end coil 84 can be increased, and a clearance X2 can be provided between portions of the wire constituting the front end coil 84. As a result, the flexibility is improved, and the rear end 84a of the front end coil 84, in which the clearance X2 is provided between portions of the wire, easily catches a gap 10a of the stent 10. In this way, the partially-deployed stent 10 can be retrieved into the catheter 2 more reliably.

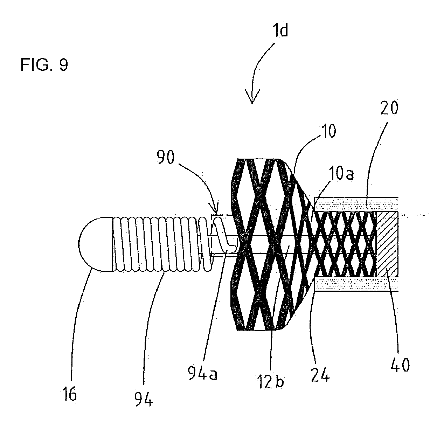

Finally, a pusher guidewire 1d according to a fifth embodiment will be described with reference to Fig. 9. It should be noted that, in Fig. 9, similarly to Figs. 1A to 5B, the left side corresponds to the front end side (distal side) inserted into a body, and the right side corresponds to the rear end side (proximal side, base end side) operated by an operator such as a doctor. Fig. 9 is an enlarged cross-sectional view of section B shown in Fig. 2A.

The pusher guidewire 1d is different from the pusher guidewire 1 shown in Figs. 1A to 5B in that a rear end 94a of a front end coil 94 functions as a latching member 90. The latching member 90 has a shape of a hook (see Fig. 9). This can reduce the risk that the rear end 94a of the front end coil 94 that caught a gap 10a of the stent 10 slips off the gap 10a of the stent 10 when the core shaft 12 is rotated in the circumferential direction (the direction of arrow 70) with the rear end 94a of the front end coil 94 catching the gap 10a of the stent 10 (see Figs. 4A and 4B). The shape of the latching member 90 is not limited to any specific shape. For example, the latching member 90 may have a bulged shape.

When positioning the front end opening 24 of the catheter 2 at the target site, the catheter 2 and the pusher guidewire 1, 1a, 1b, 1c, 1d may be delivered together with the pusher guidewire 1, 1a, 1b, 1c, 1d inserted into the catheter 2 in advance (the state of Figs. 1A and 1B), or the pusher guidewire 1, 1a, 1b, 1c, 1d may be inserted from the rear end opening 22 of the catheter 2 after positioning only the catheter 2 at the target site in advance.

As described above, in the pusher guidewire 1, 1a, 1b, 1c, 1d, the rear end 14a, 64a, 74a, 84a, 94a of the front end coil 14, 64, 74, 84, 94 is a free end that is not fixed to the first front end portion 12a of the core shaft 12. Therefore, by pulling the core shaft 12 toward the rear end, the free rear end 14a, 64a, 74a, 84a, 94a of the front end coil 14, 64, 74, 84, 94 catches a gap 10a of the partially-deployed stent 10. By rotating the core shaft 12 in this state, the front end portion 11 of the stent 10 deployed from the front end opening 24 of the catheter 2 can be wrapped around the outer circumference of the front end coil 14, 64, 74, 84, 94. By further pulling the core shaft 12 toward the rear end with the stent 10 thus wrapped, the partially-deployed stent 10 can be retrieved into the catheter 2. As a result, the inner wall of a normal blood vessel can be prevented from being damaged by the stent 10.

### [Reference Signs List]

- 1, 1a, 1b, 1c, 1d: pusher guidewire
- 2: catheter
- 10: stent
- 12: core shaft
- 14, 64, 74, 84, 94: front end coil
- 14a, 64a, 74a, 84a, 94a: rear end (free end) of front end coil
- 16: front end tip
- 18: rear end coil
- 20: tubular body
- 22: rear end opening
- 24: front end opening
- 30, 32: joining member
- 40: pusher
- 50: arrow (toward front end)
- 60: arrow (toward rear end)
- 70: arrow (rotation direction)
- 80: arrow (toward rear end)
- 90: latching member

## Claims

1. A pusher guidewire (1, 1a, 1b, 1c, 1d) for delivering a stent (10) to a target site, comprising:
a core shaft (12);
a coil (14, 64, 74, 84, 94) covering a front end portion of the core shaft; and
a joining member (30) joining the front end portion of the core shaft and the coil,
**characterized in that** a rear end (14a, 64a, 74a, 84a, 94a) of the coil is a free end that is not fixed to the core shaft.

2. The pusher guidewire according to claim 1,
**characterized in that** an outer diameter of the coil is larger from the joining member to the rear end of the coil (D2) than from a front end of the coil to the joining member (D1).

3. The pusher guidewire according to claim 1 or 2,
**characterized in that** a wire diameter of a wire constituting the coil is larger from the joining member to the rear end of the coil (D4) than from the front end of the coil to the joining member (D3).

4. The pusher guidewire according to any of claims 1 to 3,
**characterized in that** a winding pitch of the coil is larger from the joining member to the rear end of the coil (X2) than from the front end of the coil to the joining member (X1).

5. The pusher guidewire according to any of claims 1 to 4,
**characterized in that** the rear end of the coil is provided with a latching member (90).
